# EUROPEAN PATENT APPLICATION

(11) **EP 4 782 460 A1**
(43) Date of publication of application: **29.07.2026**
(21) Application number: 24868177.7
(22) Date of filing: 10.09.2024
(51) Int. Cl.: C07K 7/08, A61K 31/513, A61K 31/7072, A61K 31/7088, A61K 38/02, A61K 47/62, A61K 48/00, A61P 31/12, A61P 35/00, C07K 19/00, C12N 5/07, C12N 15/87

(54) **PEPTIDE FRAGMENT AND USE THEREOF**

(30) Priority: 19.09.2023 JP 2023151637
(71) Applicant: Toagosei Co., Ltd., Minato-ku Tokyo 105-8419 (JP)
(72) Inventor: BAILEYKOBAYASHI, Nahoko, Tokyo 1058419 (JP); YOSHIDA, Tetsuhiko, Tokyo 1058419 (JP)
(74) Representative: Bals & Vogel Patentanwälte PartGmbB
(86) International application number: PCT/JP2024/032445
(87) International publication number: WO 2025/063100

(57) **Abstract**

A novel peptide fragment having cell membrane permeability is provided. The peptide fragment disclosed herein includes any of the following amino acid sequences: KKRTLRKSARKKR (SEQ ID No.: 1); KKRTLRKSASARKKR (SEQ ID No.: 2); KKRTLRKSNSNRKKR (SEQ ID No.: 3); KKRTLRKSASNRKKR (SEQ ID No.: 4); KKRTLRKSNSARKKR (SEQ ID No.: 5); KKRTLRKSNSASARKKR (SEQ ID No.: 6); KKRTLRKSASASNRKKR (SEQ ID No.: 7); KKRTLRKSNSNSNRKKR (SEQ ID No.: 8); and KKRTLRKSNSNSNSNRKKR (SEQ ID No.: 9).

## Description

### [Technical Field]

The present disclosure relates to a peptide fragment and use thereof. The present application claims priority on the basis of Japanese Patent Application No. 2023-151637 filed on September 19, 2023, and the entire content of this application is incorporated into the description of the present application by reference.

### [Background Art]

Conventionally, polypeptides and other foreign substances have been transferred to the inside of the cells of humans and other mammals, etc., (eukaryotic cells) to change the characteristics of the cells (as well as the tissues and organs including the cells) or to improve and enhance the function of the cells.

In the aforementioned art, for example, a peptide fragment with cell membrane permeability (cell penetrating peptide, CPP) that can transfer the foreign substance to the inside of a cytoplasm through a cell membrane from outside the cell has been used. Japanese Patent Application Publication No. 2022-047613 and WO2011/013700 disclose one example of a construct for transferring a foreign substance including a cell penetrating peptide and a foreign substance that is bonded to the peptide.

### [Citation List]

### [Patent Literatures]

[Patent Literature 1] Japanese Patent Application Publication No. 2022-047613
[Patent Literature 2] WO2011/013700

### [Summary of Invention]

### [Technical Problem]

The cell penetrating peptide can be used for techniques of, for example, drug delivery systems and the like; therefore, the development of various cell penetrating peptides has been demanded.

The present disclosure has been made in view of the above circumstances, and it is an object to provide a peptide fragment and a technique to use the peptide fragment.

### [Solution to Problem]

The present disclosure provides a peptide fragment (synthetic peptide) having cell membrane permeability. One aspect of the peptide fragment disclosed herein is a peptide fragment including an A part, a B part, and a C part from an N-terminus of a peptide, in which the A part is an amino acid sequence set forth in SEQ ID No.: 11 (KKRTLRK), the C part is an amino acid sequence set forth in SEQ ID No.: 12 (RKKR), and the B part is positioned between the A part and the C part and is an amino acid sequence in which one unit, two units, three units, or four units are arranged from the group consisting of SA and SN (however, a sequence in which the B part includes only SN is excluded).

The inventor has examined a construct for transferring a foreign substance disclosed in Patent Literature 1 mentioned above in order to transfer a foreign substance to the inside of a cytoplasm through a cell membrane from outside a eukaryotic cell with higher efficiency. For example, manufacture of a construct with higher cell membrane permeability has been tried by performing the substitution of various amino acid residues for an amino acid sequence (KKRTLRKSNRKKR) set forth in SEQ ID No.: 10. As a result, it has been found out that the amino acid sequence (KKRTLRKSNRKKR) can be divided into the A part (SEQ ID No.: 11: KKRTLRK), the B part, and the C part (SEQ ID No.: 12: RKKR). In addition, by performing the substitution of the amino acid sequence of the B part positioned between the A part and the C part, the peptide fragment can be completed without deteriorating the cell permeability.

The substitution of the amino acid residue for the B part is not simple conservative amino acid replacement (for example, a sequence in which a basic amino acid residue is replaced by another basic amino acid residue). As a result of trial and error by the present inventor, it has been found out that, when the B part includes one to four units of an SA unit or an SN unit, the cell permeability is achieved without increasing cytotoxicity.

The peptide fragment with such a structure has the cell membrane permeability. Thus, a construct including the peptide fragment is transferred from outside a cell into the cell (into at least a cytoplasm).

In the peptide fragment of one aspect disclosed herein, the B part is the amino acid sequence in which an amino acid sequence set forth in SA is continuous for one unit, two units, or three units. In such a peptide fragment, the cell membrane permeability can be improved drastically.

The peptide fragment of one aspect disclosed herein includes any of the following amino acid sequences:
KKRTLRKSARKKR (SEQ ID No.: 1);
KKRTLRKSASARKKR (SEQ ID No.: 2);
KKRTLRKSNSNRKKR (SEQ ID No.: 3);
KKRTLRKSASNRKKR (SEQ ID No.: 4);
KKRTLRKSNSARKKR (SEQ ID No.: 5);
KKRTLRKSNSASARKKR (SEQ ID No.: 6);
KKRTLRKSASASNRKKR (SEQ ID No.: 7);
KKRTLRKSNSNSNRKKR (SEQ ID No.: 8); and
KKRTLRKSNSNSNSNRKKR (SEQ ID No.: 9).
In such a peptide fragment, the cell permeability is achieved without increasing the cytotoxicity

The present disclosure provides a construct for transferring a foreign substance (hereinafter also referred to as "construct" simply) that can transfer a target foreign substance from outside a eukaryotic cell to the inside of the cell. One aspect of the construct disclosed herein includes a peptide fragment, and a foreign substance that is bonded to an N-terminus and/or a C-terminus of the peptide fragment, in which the peptide fragment includes an A part, a B part, and a C part from the N-terminus, the A part is an amino acid sequence set forth in SEQ ID No.: 11 (KKRTLRK), the C part is an amino acid sequence set forth in SEQ ID No.: 12 (RKKR), and the B part is positioned between the A part and the C part and is an amino acid sequence in which one unit, two units, three units, or four units are arranged from the group consisting of SA and SN (however, a sequence in which the B part includes only SN is excluded). With such a construct, the foreign substance can be transferred from outside the eukaryotic cell to the inside of the cell (for example, into at least a cytoplasm).

In one aspect of the construct disclosed herein, the B part is the amino acid sequence in which an amino acid sequence set forth in SA is continuous for one unit, two units, or three units. Thus, the foreign substance can be transferred more certainly from outside the eukaryotic cell to the inside of the cell.

In one aspect of the construct disclosed herein, the peptide fragment includes any of the following amino acid sequences:
KKRTLRKSARKKR (SEQ ID No.: 1);
KKRTLRKSASARKKR (SEQ ID No.: 2);
KKRTLRKSNSNRKKR (SEQ ID No.: 3);
KKRTLRKSASNRKKR (SEQ ID No.: 4);
KKRTLRKSNSARKKR (SEQ ID No.: 5);
KKRTLRKSNSASARKKR (SEQ ID No.: 6);
KKRTLRKSASASNRKKR (SEQ ID No.: 7);
KKRTLRKSNSNSNRKKR (SEQ ID No.: 8); and
KKRTLRKSNSNSNSNRKKR (SEQ ID No.: 9).
With such a construct, the foreign substance is transferred from outside the eukaryotic cell to the inside of the cell.

In one aspect of the construct disclosed herein, the foreign substance is at least one kind of organic compound selected from the group consisting of a polypeptide, a nucleic acid, a dye, and a drug. Thus, an organic substance with various functions can be transferred to the inside of the eukaryotic cell.

In one aspect of the construct disclosed herein, the foreign substance is disposed at the C-terminus of the peptide fragment. Thus, the construct can be transferred to the inside of the eukaryotic cell efficiently.

The present disclosure provides a method for transferring a target foreign substance in vitro or in vivo from outside a eukaryotic cell to the inside of at least a cytoplasm of the cell. One aspect of the method disclosed herein includes: (1) a step of preparing the construct disclosed herein; and (2) a step of supplying the construct into a sample including a target eukaryotic cell. Thus, the target foreign substance can be transferred to the inside of the eukaryotic cell.

In one aspect of the method disclosed herein, the eukaryotic cell to which the construct is to be transferred is a mammalian cell. Thus, the target foreign substance can be transferred to the inside of the mammalian cell.

### [Brief Description of Drawings]

Fig. 1 is a graph showing mean fluorescence intensity (MFI) in each example, which is obtained by culturing with FAM or constructs according to Examples 1 to 3 and Comparative Examples 1 and 2 added to a culture solution of NSC-34 cells, and then analyzing the cells with a flow cytometer.
Fig. 2 is a graph showing the mean fluorescence intensity (MFI) in each example, which is obtained by culturing with FAM or constructs according to Examples 4 to 9 and Comparative Example 3 added to the culture solution of NSC-34 cells, and then analyzing the cells with the flow cytometer.

### [Description of Embodiments]

### <Defmition>

Some embodiments of the art disclosed herein will be described below. Matters that are other than matters particularly mentioned in the present specification and that are necessary for the implementation of the present art (for example, general matters related to chemical synthesis methods for peptide, cell culture techniques, and preparation of constructs containing peptides or nucleic acids as a component) can be grasped as design matters of those skilled in the art based on the prior art in the fields such as cell engineering, physiology, medical science, pharmacology, organic chemistry, biochemistry, genetic engineering, protein technology, molecular biology, and genetics. Moreover, the patent technology disclosed herein can be carried out on the basis of the contents disclosed in this specification and common technical knowledge in the fields. In the following description, the amino acids may be represented by single letter codes based on the nomenclature for amino acid residues in the IUPAC-IUB guidelines. Note that the term "amino acid residue" in this specification encompasses N-terminus amino acids and C-terminus amino acids of a peptide chain unless otherwise stated specifically.

In this specification, the term "synthetic peptide" refers to a peptide (peptide fragment) whose peptide chain alone does not stably exist in nature. The synthetic peptide is manufactured by artificial chemical synthesis or biosynthesis (that is, production based on genetic engineering) and can stably exist in a predetermined composition. Here, the term "peptide" refers to an amino acid polymer (including a dimer, a trimer, and an oligomer) having one or a plurality of peptide bonds and is not limited by the number of amino acid residues.

The amino acid residue of the peptide or protein in this specification may be either an L-form or a D-form unless otherwise stated in particular. That is to say, each of the amino acid residues included in the amino acid sequences according to the scope of claims includes an aspect of the L-form or the D-form. Note that the amino acid sequence in this specification always represents the N-terminus on the left side and the C-terminus on the right side.

### <Peptide fragment>

A peptide fragment disclosed herein is a peptide fragment that includes an A part, a B part, and a C part from an N-terminus of the peptide, in which the A part is an amino acid sequence set forth in SEQ ID No.: 11 (KKRTLRK), the C part is an amino acid sequence set forth in SEQ ID No.: 12 (RKKR), and the B part is a synthetic peptide that is positioned between the A part and the C part and includes an amino acid sequence in which one unit, two units, three units, or four units are arranged from the group consisting of SA and SN (however, a sequence in which the B part includes only SN is excluded).

The peptide fragment disclosed herein can be divided into the A part, the B part, and the C part from the N-terminus. The B part includes the amino acid sequence in which an arbitrary number of one to four units of the SA unit or the SN unit are arranged by arbitrary combinations.

A preferable embodiment of the peptide fragment disclosed herein is a synthetic peptide that includes KKRTLRKSARKKR (SEQ ID No.: 1), KKRTLRKSASARKKR (SEQ ID No.: 2), KKRTLRKSNSNRKKR (SEQ ID No.: 3), KKRTLRKSASNRKKR (SEQ ID No.: 4), KKRTLRKSNSARKKR (SEQ ID No.: 5), KKRTLRKSNSASARKKR (SEQ ID No.: 6), KKRTLRKSASASNRKKR (SEQ ID No.: 7), KKRTLRKSNSNSNRKKR (SEQ ID No.: 8), or KKRTLRKSNSNSNSNRKKR (SEQ ID No.: 9). In one aspect, the peptide fragment disclosed herein includes any of the amino acid sequences set forth in SEQ ID No.: 1 to SEQ ID No.: 9. The peptide fragments including the amino acid sequences set forth in SEQ ID No.: 1 to SEQ ID No.: 9 have cell membrane permeability, and are transferred efficiently from outside a cell membrane of a eukaryotic cell to the inside. Therefore, a construct including the peptide fragment disclosed herein can be transferred efficiently from outside the cell membrane of the eukaryotic cell to the inside.

The number of amino acid residues of the peptide fragment disclosed herein is not limited as long as the cell membrane permeability is not deteriorated. The number of amino acid residues of the peptide fragment can be, for example, 19 or less, 17 or less, 15 or less, or 13 or less.

In the peptide fragment disclosed herein, excluding the B part, the amino acid sequence of the A part set forth in SEQ ID No.: 11 (KKRTLRK) and the amino acid sequence of the C part set forth in SEQ ID No.: 12 (RKKR) include typically the same sequence. In addition to the same sequence, however, a modified sequence may be included unless the cell membrane permeability is deteriorated. Here, the "modified sequence" corresponds to an amino acid sequence (modified amino acid sequence) formed by the substitution, deletion, and/or addition (insertion) of one or several (for example, two) amino acid residues. That is to say, such a slightly modified sequence can be easily used on the basis of the technical information disclosed herein, and is encompassed in the peptide fragment as a technical concept disclosed herein. Typical examples of the modified sequence in this specification include, for example, a sequence produced by so-called conservative amino acid replacement in which one or two amino acid residues are conservatively replaced, a sequence in which one, two, or three amino acid residues are added (inserted) to or deleted from a predetermined amino acid sequence, and the like. Typical examples of the conservative amino acid replacement include, for example, a sequence in which a basic amino acid residue is replaced by another basic amino acid residue (for example, mutual replacement between a lysine residue and an arginine residue), and the like.

In the peptide fragment disclosed herein, the B part includes the amino acid sequence in which one to four units of the SA unit or the SN unit are arranged. For example, in the amino acid sequence set forth in SEQ ID No.: 1, the B part includes one SA unit. In the amino acid sequence set forth in SEQ ID No.: 2, the B part includes two SA units. In the amino acid sequence set forth in SEQ ID No.: 3, the B part includes two SN units. In the amino acid sequence set forth in SEQ ID No.: 4, the B part includes one SA unit and one SN unit from the N-terminus. In the amino acid sequence set forth in SEQ ID No.: 5, the B part includes one SN unit and one SA unit from the N-terminus. In the amino acid sequence set forth in SEQ ID No.: 6, the B part includes one SN unit and two SA units from the N-terminus. In the amino acid sequence set forth in SEQ ID No.: 7, the B part includes two SA units and one SN unit from the N-terminus. In the amino acid sequence set forth in SEQ ID No.: 8, the B part includes three SN units. In the amino acid sequence set forth in SEQ ID No.: 9, the B part includes four SN units.

The construct disclosed herein includes the peptide fragment disclosed herein and a foreign substance. The foreign substance is bonded to an N-terminus and/or a C-terminus of the peptide fragment.

### <Linker>

In some aspects, the construct is designed and constructed in such a way that the foreign substance is directly or indirectly bonded (coupled) to the N-terminus and/or the C-terminus of the peptide fragment. In the case where the peptide fragment and the foreign substance are bonded together indirectly, for example, a linker is disposed between the peptide fragment and the foreign substance. The linker does not need to be limited in particular. Moreover, a method of directly or indirectly bonding the peptide fragment and the foreign substance is not limited in particular and various conventionally known scientific methods can be employed.

The peptidic linker can be constructed by one, or two or more amino acid residues. The number of amino acid residues included in the peptidic linker is not limited in particular. The number of amino acid residues included in the peptidic linker is, for example, one or more or two or more. In addition, the number of amino acid residues included in the peptidic linker is, for example, ten or less or five or less. In some aspects, the amino acid sequence included in the peptidic linker is a flexible amino acid sequence that does not cause steric hindrance. Such a peptidic linker can include, for example, glycine, alanine, and/or serine. In some aspects, moreover, the peptidic linker can include β-alanine.

Examples of the non-peptidic linker include an alkyl linker, a polyethylene glycol (PEG) linker, amino hexanoyl spacer, and the like, although there is no particular limitation. In addition, a crosslinking agent including a homo-bifunctional group or a hetero-bifunctional group may be used as the non-peptidic linker. As a general functional group included in the crosslinking agent, N-hydroxysuccinimide activated ester (NHS ester), maleimide, azide, iodoacetamide, or the like is given. Among these, NHS ester can be bonded with amine through efficient reaction at pH more than or equal to the neutrality. In addition, maleimide can be bonded through selective reaction with an SH group. For example, in a case of using the crosslinking agent including maleimide, a cysteine residue is added as a linker to the N-terminus or the C-terminus of the peptide fragment or the peptide fragment including a cysteine residue at the N-terminus or the C-terminus is prepared; thus, the bonding between the peptide fragment and the crosslinking agent can be achieved easily.

Preferred crosslinking agents including the homo-bifunctional group include N-hydroxysuccinimide (NHS), disuccinimidyl suberate (DSS), bis(sulfosuccinimidyl) suberate (BS³), dithiobis(succinimidyl propionate) (DSP), dithiobis(sulfosuccinimidyl propionate) (DTSSP), ethylene glycol bis(succinimidyl succinate) (EGS), ethylene glycol bis(sulfosuccinimidyl succinate) (sulfo-EGS), disuccinimidyl tartrate (DST), disulfosuccinimidyl tartrate (sulfo-DST), and the like. In particular, bis(sulfosuccinimidyl) suberate (BS³) can be preferably used.

Preferred crosslinking agents including the hetero-bifunctional group include N-(6-maleimidocaproyloxy)succinimide (EMCS), m-maleimidobenzoyl-N-hydroxysuccinimide ester (MBS), succinimidyl4-[maleimidophenyl]butylate (SMPB), succinimidyl4-(maleimidomethyl)cyclohexane-1-carboxylate (SMCC), N-(y-maleimidebutyloxy)succinimide ester (GMBS), m-maleimidopropionic acid-N-hydroxysuccinimide ester (MPS), N-succinimidyl(4-iodoacetyl)aminobenzoate (STAB), and the like. In particular, EMCS and MBS including NHS ester and maleimide in a reactive functional group can be preferably used.

### <Foreign substance>

The foreign substance is not limited in particular as long as having the molecular size and chemical properties that can be transferred to the inside of the eukaryotic cell, and can be, for example, an organic compound or an inorganic compound. Examples of an organic substance include an amino acid, a polypeptide, a nucleic acid, a dye, a drug, and the like. One kind of the foreign substance may be included alone, or two or more kinds thereof may be included. In the case where two or more kinds of the foreign substances are included, the two or more kinds of the foreign substances may be bonded to the N-terminus or the C-terminus of the peptide fragment. In addition, one kind, or two or more kinds of the foreign substances may be bonded to each of the N-terminus and the C-terminus of the peptide fragment.

In the case where the foreign substance is a polypeptide, the polypeptide (amino acid sequence) to be employed is not limited in particular. In this specification, the term "polypeptide" refers to a polymer (including a dimer, a trimer, and an oligomer) having a structure in which a plurality of amino acids are bonded by a peptide bond. The polypeptide is not limited by the number of peptide bonds (that is, the number of amino acid residues). As the polypeptide, for example, the polypeptide with relatively many amino acid residues, such as a peptide including about 2 to 100 amino acid residues, a polypeptide including about 100 to 300 amino acid residues, or proteins (typically, a polymer compound including 300 or more amino acid residues) can also be employed as the foreign substance. In this field, the polypeptides and the proteins are not distinguished strictly. The upper limit of the number of amino acid residues included in the polypeptide is not limited in particular; however, from the viewpoint of facilitating the synthesis (biosynthesis or chemical synthesis), for example, the upper limit is preferably 1000 or less, 600 or less, 500 or less, 400 or less, or 300 or less.

The polypeptide can be a mature form or precursor (including pro-forms and prepro-forms) of a polypeptide involved in a function such as the development, differentiation, growth, malignant transformation, homeostasis, or regulation of metabolism in various cells and tissues (organs). Moreover, the polypeptide may be a polypeptide with a heretofore unknown function. In accordance with the art disclosed herein, the unknown function of the polypeptide in the cell (in a biological tissue) can be elucidated.

For example, if the eukaryotic cell that is the target to which the foreign substance is to be transferred is a stem cell of a mammalian (human or other mammalian) cell, it is preferable to use a mature form or precursor of a polypeptide with various biological activities related to the differentiation induction of the stem cells. Note that the term "stem cell" encompasses somatic stem cells, embryonic stem cells, and induced pluripotent stem cells (iPS cells). Moreover, when the eukaryotic cell to which the foreign substance is to be transferred is a cancer cell (tumor cell), it is preferable to use various polypeptides involved in the induction of apoptosis of the cancer cell (tumor cell). Moreover, when the eukaryotic cell that is the target of transfer is a bacteria-infected cell or a virus-infected cell, it is preferable to use various polypeptides involved in inducing apoptosis of these infected cells, a polypeptide that can inhibit the increase of cells or virus in the infected cells, or a polypeptide that can inhibit the expansion of the bacteria or virus infection from the infected cells. Furthermore, similarly to the peptide fragment, the polypeptide as the foreign substance may include a modified amino acid sequence that is formed by the replacement, deletion, and/or addition (insertion) of one or several amino acid residues as long as that function is kept.

In the case where the foreign substance is the polypeptide, for example, a peptide chain is designed so as to include an amino acid sequence that forms the polypeptide and an amino acid sequence that forms the peptide fragment, and by biosynthesis or chemical synthesis of such a peptide chain, a construct in which the polypeptide is directly bonded to the peptide fragment can be manufactured.

The kind of the nucleic acid to be employed as the foreign substance is not limited in particular and for example, can include so-called nucleic acid medicine. In this specification, the term "nucleic acid" refers to a polymer of nucleotide, and encompasses DNA, RNA, and DNA/RNA hybrid (also called DNA-RNA chimera) including both DNA and RNA. In addition, "the nucleic acid" is not limited by the number of bases. The nucleic acid may be either single-stranded (including hair pin type single-stranded) or double-stranded. Moreover, the nucleotide included in the nucleic acid may include a modifying group in a phosphate part, a saccharide part, or a base part. The kind of the nucleic acid medicine is not limited in particular and for example, siRNA, miRNA, anti-sense, aptamer, decoy, ribozyme, CpG oligo, or the like is given.

The kind of the dye to be employed as the foreign substance is not limited in particular. The dye can include various fluorescent compounds such as FAM and FITC. When the construct includes the dye, the transfer of the construct can be evaluated easily. For example, the efficiency of transferring the construct to the eukaryotic cell can be evaluated using a microscope observation (for example, fluorescence microscope observation), a flowcytometry, an immunochemical method (for example, Western Blot, immunocytochemistry, or the like), or the like.

The kind of the drug to be employed as the foreign substance is not limited in particular. The drug can include various organic compounds, for example an antitumor drug including a nucleic acid-based antitumor drug such as 5-fluorouracil (5FU), an antiviral drug such as azidothymidine (AZT), or the like.

### <Construct>

In the construct, in the case where the foreign substance is bonded to the C-terminus of the peptide fragment, an α-amino group of the amino acid residue of the N-terminus of the peptide fragment is preferably acetylated. Although the detailed mechanism is not clear, since the α-amino group of the amino acid of the N-terminus in most of the proteins in the eukaryotic cell is subjected to the acetylation modification, such a structure can improve the stability of the construct in the cells.

In the construct, the amino acid residue of the C-terminus is preferably amidated. The structural stability (for example, protease resistance) of the construct as described above in the cytoplasm or nucleus can be improved by amidation of a carboxyl group of the amino acid residue (typically, a C-terminal amino acid residue of a peptide chain). In addition, the amidation of the carboxyl group improves the hydrophilicity of the construct, so that the solubility of this construct in an aqueous solvent can be improved. Examples of such an aqueous solvent include water, various buffer solutions, physiological saline (for example, PBS), a cell culture solution, and the like. For example, in the case of the construct in which the foreign substance is bonded to the N-terminus of the peptide fragment, a carboxyl group of the amino acid residue of the C-terminus of the peptide fragment is preferably amidated. For example, in the case of the construct in which the foreign substance is bonded to the N-terminus of the peptide fragment, a carboxyl group of the amino acid residue of the C-terminus of the peptide fragment is preferably amidated. Moreover, in a case where the foreign substance is the polypeptide and this polypeptide is bonded to the C-terminus of the peptide fragment, it is preferable to amidate the carboxyl group of the C-terminal amino acid residue of the polypeptide.

In the peptide fragment or the construct, the peptide part (polypeptide, peptide fragment, and peptidic linker as the foreign substance) can easily be manufactured by, for example, a general chemical synthesis method. For example, either a conventionally known solid phase or liquid phase synthesis method can be used. A solid phase synthesis method using Boc (t-butyloxycarbonyl) or Fmoc (9-fluoroenylmethoxycarbonyl) as a protecting group for an amino group is preferred. In other words, the aforementioned peptide part with the desired amino acid sequence and modifications (N-terminal acetylation, C-terminal amidation, etc.) can be synthesized by a solid phase synthesis method using a commercially available peptide synthesizer. Note that only a part of the peptide chain may be synthesized by the aforementioned method and for example, the peptide chain including only the peptide fragment or including the peptide fragment and the peptidic linker part may be synthesized.

The peptide part of the peptide fragment or the construct may be manufactured by biosynthesis in accordance with a genetic engineering method. In other words, a polynucleotide (typically, DNA) of a nucleotide sequence (including the ATG start codon) that codes for the desired amino acid sequence is synthesized. Then, a recombinant vector with a genetic construct for expression that includes the synthesized polynucleotide (DNA) and various regulatory elements (including a promoter, ribosome binding site, terminator, enhancer, and various cis-elements for controlling the level of expression) to express the amino acid sequence in a host cell is configured in accordance with the host cell. Using the general techniques, this recombinant vector is transferred to a predetermined host cell (for example, yeast, insect cell, or plant cell), and the host cell, or a solid or tissue containing the cell is cultured under a predetermined condition. The target peptide can be produced in the cell thereby. Furthermore, the target peptide part can be obtained by isolating the peptide part from the host cell (or from a culture medium if it is secreted) and if necessary, refolding and purifying the peptide part, for example. Note that the method for configuring the recombinant vector and the method for transferring the configured recombinant vector to the host, etc., may utilize methods conventionally used in the fields without modification, and because those methods themselves are not a characterizing feature of the present art, the detailed explanation thereof is omitted.

For example, in the manufacture of the peptide part of the construct, a fusion protein expression system can be used for efficient, large volume production in host cells. For example, the gene (DNA) coding for the amino acid sequence of the target polypeptide is obtained by chemical synthesis, and the synthesized gene is transferred to a suitable site in a suitable fusion protein expression vector (for example, a glutathione S-transferase (GST) fusion protein expression vector such as the pET series provided by Novagen and the pGEX series provided by Amersham Biosciences). Then, the host cells (typically E. coli) are transformed by the vector. The resulting transformant is cultured to prepare the target fusion protein. Next, the protein is extracted and purified. Then, the resulting purified fusion protein is cleaved by a predetermined enzyme (protease) and the freed target peptide fragment (i.e., the designed artificial peptide) is recovered by a method such as affinity chromatography. The target construct (artificial polypeptide) can be manufactured using this kind of conventionally known fusion protein expression system (for example, the GST/His system provided by Amersham Biosciences can be utilized).

Template DNA for use in a cell-free protein synthesis system (i.e., a synthetic gene fragment containing a nucleotide sequence coding for the amino acid sequence of the peptide part for the construct) can be configured, and in-vitro synthesis of the target peptide part can be carried out by employing a so-called cell-free protein synthesis system using various compounds necessary for synthesis of the peptide part (ATP, RNA polymerase, amino acids, etc.). References concerning a cell-free protein synthesis system include, for example, the papers by Shimizu et al. (Shimizu et al., Nature Biotechnology, 19, 751-755 (2001)) and Madin et al. (Madin et al., Proc. Natl. Acad. Sci. USA, 97(2), 559-564 (2000)). At the time of the filing of the present application, there are already many companies carrying out polypeptide production on consignment on the basis of the technology disclosed in these documents, and cell-free protein synthesis kits are commercially available (for example, obtainable from Cell Free Sciences Co., Ltd. in Japan).

A single-stranded or double-stranded polynucleotide including the nucleotide sequence coding for the peptide part of the construct and/or the nucleotide sequence that is complementary to the above-described nucleotide sequence can be manufactured (synthesized) easily by the conventionally known method. For example, by selecting the codon that corresponds to the amino acid residue included in the peptide part, the nucleotide sequence corresponding to such an amino acid sequence is easily determined and provided. Once the nucleotide sequence is determined, the polynucleotide (single strand) corresponding to the desired nucleotide sequence can be easily obtained using a DNA synthesizer or the like. Furthermore, by using the obtained single-stranded DNA as a template, the target double-stranded DNA can be obtained with various enzymatic synthesizing means (typically, PCR). Moreover, the polynucleotide may be either in a DNA form or an RNA (mRNA, etc.) form. DNA can be provided as a double strand or a single strand. In the case of providing DNA as a single strand, the strand may be either a code strand (sense strand) or a non-code strand (anti-sense strand) with the sequence complementary to that code strand. The polynucleotide obtained in this manner can be used as a material for configuring a recombination gene (expression cassette) for peptide production in various host cells or a cell-free protein synthesis system as described above. Note that the nucleic acid as the foreign substance can also be prepared similarly to the aforementioned manufacturing method.

The construct disclosed herein can be suitably used as an effective component of the composition for the usage based on the function of the foreign substance. Note that the construct may be in a salt form unless the function of the foreign substance is lost. For example, an acid addition salt that can be obtained by carrying out an addition reaction with a normally used inorganic or organic acid in accordance with a conventional method can be used. Thus, "the construct" according to the present specification and the scope of claims can encompass the construct in such a salt form.

The construct can be used as an effective component of the composition that can contain various carriers that are allowable in terms of medical (pharmaceutic) perspectives in accordance with the usage mode. As the carrier, for example, a carrier that is generally used in the peptide medical fields such as a diluent or an excipient is preferable. As such a carrier, typically, water, a physiological buffer, and various organic solvents are given, although depending as appropriate on the usage or mode of the construct for transferring a foreign substance. Moreover, the carrier can be an alcohol (such as ethanol) aqueous solution with a suitable concentration, glycerol, or non-drying oil such as olive oil, or may be liposome. As a secondary component that can be contained in a pharmaceutical composition, various filling agents, thickening agents, bonding agents, moisture adding agents, surfactants, dyes, flavoring agents, and the like are given.

### <Composition>

The mode of the composition is not limited in particular. For example, modes of liquids, suspensions, emulsions, aerosols, foams, granules, powders, tablets, capsules, and ointments are given. Moreover, for the use in injection or the like, a lyophilized product or granulated product to prepare a drug solution by being dissolved in physiological saline or a suitable buffer (e.g., PBS), etc., just before use can be formed. The conventionally known methods may be used for the processes themselves to prepare various modes of medicines (compositions) from the construct (main component) and various carriers (secondary components) as materials. A detailed explanation of such a manufacturing process itself is omitted herein because it is not a characterizing feature of the present art. For example, Comprehensive Medicinal Chemistry, edited by Corwin Hansch, Pergamon Press, 1990, can be noted as a source of detailed information concerning formulations.

In some aspects, the composition includes the peptide fragment disclosed herein and the foreign substance that is not bonded to the N-terminus or the C-terminus of the peptide fragment. In such aspects, for example, the peptide fragment can form a complex with the foreign substance by an electric interaction or a molecular interaction. Such a complex can be transferred easily to the inside of the eukaryotic cell, making it possible to transfer the foreign substance to the inside of a eucaryote efficiently. In a preferred aspect, the foreign substance included in the composition is a substance that is negatively charged (for example, nucleic acid). The peptide fragment disclosed herein contains a high proportion of basic amino acid and is positively charged; therefore, the ratio thereof to the negatively charged foreign substance or the like is not limited in particular. For example, the ratio of the peptide fragment to the foreign substance in terms of moles may be 5 to 100, and is preferably 40 to 60.

### <Use of construct>

The present disclosure provides a method for transferring a target foreign substance from outside a eukaryotic cell to the inside of the cell in vivo or in vitro using the construct disclosed herein. Here, "the inside of the eukaryotic cell" refers to the inside of the eukaryotic cell that is surrounded by a cell membrane, and encompasses, for example, cytoplasm, cytosol, and organelle (for example, nuclear, mitochondria, endoplasmic reticulum, microtubule, lysosome, Golgi, and the like). In the method disclosed herein, for example, the construct can be transferred to at least the cytoplasm of the eukaryotic cell (further to the organelle).

Roughly speaking, the method disclosed herein includes the following steps (1) and (2):
(1) a step of preparing a construct disclosed herein; and
(2) a step of supplying the construct into a sample including a target eukaryotic cell. The method disclosed herein can further include, after the step (2), a step (3) of incubating the sample to which the construct has been supplied, thereby transferring the construct to the inside of the eukaryotic cell in the sample.

The term "eukaryotic cell" encompasses, in vivo, various tissues, viscera, organs, blood, lymph, and the like, for example. The term "eukaryotic cell" encompasses, in vitro, various cell clusters, tissues, viscera, organs, blood, lymph, cell lines, and the like extracted from a body, for example. Examples of the eukaryotic cell include cells derived from animals such as mammals, birds, fishes, amphibians, reptiles, and insects, cells derived from fungi, cells derived from plants, and the like. Preferably, the eukaryotic cell can be a cell of a human or nonhuman mammal (mammalian cell).

In the method disclosed herein, for example, a composition including the construct disclosed herein is prepared and supplied into the sample including the eukaryotic cell. Such a composition can be used in vivo in dosage and method according to the mode and intended purpose. For example, exactly a desired amount of liquid can be administered to a diseased area (for example, malignant tumor tissue, virus-infected tissue, inflamed tissue, etc.) of a patient (i.e., the body) by intravenous, intramuscular, subdermal, intradermal, or intraperitoneal injection. Alternatively, the composition in a solid mode such as a tablet or in a gel-form or aqueous jelly-form such as ointment can be applied directly to a predetermined tissue (i.e., for example, a diseased area such as an organ or a tissue including a tumor cell, virus-infected cell, inflamed cell, or the like). Further alternatively, the composition in a solid mode such as a tablet can be administered orally. In the case of the oral administration, it is preferable to perform encapsulation or apply a protection (coating) material in order to suppress decomposition by digestive enzymes in the alimentary canal.

The construct or the composition in an amount suitable for the eukaryotic cell cultured in vitro is preferably supplied to a culture solution of the target eukaryotic cell at least once. The amount per supply and the number of times of supplies are not limited in particular because they can vary depending on the conditions including the kind of eukaryotic cells to culture, the cell density (cell density at the start of culture), the passage number, the culture condition, the kind of culture medium, and the like. For example, the composition is preferably added once, twice, or more times so that the concentration of the peptide fragment in the culture solution falls within the range of about 0.05 µM or more and 100 µM or less, 0.5 µM or more and 50 µM or less, or 1 µM or more and 30 µM or less, for example. In addition, the incubating time after the construct is added is not limited in particular either because the incubating time can vary depending on the kind of eukaryotic cells and various conditions. For example, the incubating time can be 0.5 hours or more, 1 hour or more, 4 hours or more, 8 hours or more, or 20 hours or more. Moreover, the incubating condition is not limited in particular because the incubating condition can vary depending on the kind of eukaryotic cells; however, the incubation is possible in a 5% CO₂ atmosphere under 37°C, for example. Note that one example of the transferring method in vitro will be also described in test examples below.

### Examples

Several test examples concerning the art disclosed herein are described below, but it is not intended to limit the art disclosed herein to these test examples.

### <Preparation of construct>

Constructs shown in Table 1 according to Examples were prepared. In Example 1, a construct 1 formed of a peptide fragment (synthetic peptide) including the amino acid sequence set forth in SEQ ID No.: 1 in which the amino group of the lysine residue of the N-terminus is acetylated, and FAM (C₂₁H₁₂O₇: 5(6)-carboxyfluorescein, molecular weight 376.3, excitation wavelength 495 nm, fluorescence wavelength 520 nm) corresponding to fluorescent dye bonded to the C-terminus of the peptide fragment was prepared. The construct 1 was diluted with dimethyl sulfoxide (DMSO) to prepare a sample solution 1 in which the concentration of the construct 1 was 2 mM. In Examples 2 to 9 and Comparative Example 1, similarly to Example 1, constructs 2 to 10 formed of synthetic peptides including the amino acid sequences set forth in SEQ ID No.: 2 to SEQ ID No.: 10 in which the amino group of the lysine residue of the N-terminus is acetylated, and FAM corresponding to fluorescent dye bonded to the C-terminus of the synthetic peptides were prepared. The constructs 2 to 10 were diluted with DMSO to prepare sample solutions 2 to 10 in which the concentration of the constructs 2 to 10 was 2 mM. In addition, a FAM solution of 2 mM in which FAM corresponding to fluorescent dye was diluted in DMSO was prepared.

### <Evaluation of cell membrane permeability>

As the eukaryotic cells, NSC-34 cells (mouse motor neuron-like hybrid cell line) were used and the cell membrane permeability of the prepared construct was evaluated.

### (Example 1)

The NSC-34 cell was cultured in 10% fetal bovine serum (FBS)-containing Dulbecco's modified Eagle's medium (DMEM (Cat No. 044-29765, manufactured by FUJIFILM Wako Pure Chemical Corporation)), which is a culture medium. The NSC-34 cell that adhered to a culture plate was cleaned with PBS and then, a 0.25%-trypsin/EDTA solution was added thereto, and incubation was performed for three minutes at 37°C. After the incubation, the aforementioned 10% FBS-containing DMEM was added to deactivate trypsin and then, five-minute centrifugal separation was performed at 150 × g to precipitate the cells. After the supernatant generated by the centrifugal separation was removed, the 10% FBS-containing DMEM was added to the precipitate (cell pellet) to prepare a cell suspension of about 2 × 10⁵ cells/mL. The cell suspension was added by 1 mL to a well of a commercially available six-hole (well) plate (manufactured by AGC TECHNO GLASS Co., Ltd.) so as to seed the cell (about 2 × 10⁵ cells/well), and incubation was carried out for about two to three hours until the cell adhered to a bottom surface of the well. After that, the 2 mM sample solution 1 prepared as above was diluted with the 10% FBS-containing DMEM to prepare the sample solution 1 in which the concentration of the sample was 20 µM. Then, 1 mL of the 20 µM sample solution 1 was added to the well (that is, so that the concentration of the construct 1 became 10 µ and the DMSO concentration became 0.5% in the culture solution in the well). The cell was incubated for 20 hours at 37°C under a 5% CO₂ condition.

After the 20-hour incubation, the culture supernatant was removed from the well and the cells in the well were cleaned twice with 1 mL of PBS. Next, 100 µL of the 0.25% trypsin/EDTA solution was added to the well and the incubation was performed for three minutes at 37°C. After the incubation, 900 µL of the 10% FBS-containing DMEM was added to the well to deactivate the trypsin. The cell suspension in the well was transferred to a tube and the cells were collected. This tube was subjected to five-minute centrifugal separation at 4°C under a condition of 210 × g. After the centrifugal separation, the supernatant was removed and the precipitate (cell pellet) was suspended (cleaned) with 1 mL of PBS, which was followed by the centrifugal separation under the same condition as that described above. Then, the supernatant was removed and thus, the cells (cell pellet) cultured in a culture medium containing the sample were obtained.

Regarding the obtained cells (cell pellet), the cell membrane permeability was analyzed using a flow cytometer. As the flow cytometer, On-Chip Flowcytometer (manufactured by On-Chip Biotechnologies Co., LTD.) was used. For such an analysis, the obtained cell pellet was suspended with 50 µL of on-chip T buffer to prepare a cell suspension for analysis.

Using the aforementioned flow cytometer, gating based on forward scatter (FSC) and side scatter (SSC) was performed to set a gate about a cell group to be analyzed, and the fluorescence intensity was measured about the cell group in this gate. Note that the number of cells of the cell group to be analyzed was set to 10000 or more, and the analysis was performed. The fluorescence intensity was measured using a fluorescence detector FL2 of the flow cytometer (optimum detection wavelength around 543 nm) capable of detecting the fluorescence wavelength of FAM. Regarding this measurement result, the analysis was performed using commercially available analysis software "FlowJo" (manufactured by TreeStar) to obtain median fluorescence intensity (mean fluorescence intensity: MFI) of the cell group to be measured (see Table 1).

### (Examples 2 to 8 and Comparative Example 1)

The process similar to that in Example 1 was performed except that sample solutions 2 to 10 were used instead of the sample solution 1.

### (Comparative Examples 2 and 3)

In Comparative Examples 2 and 3, the process similar to that in Example 1 was performed except that the FAM solution was used instead of the sample solution 1. The concentration of this FAM solution was the same as the concentration of the sample solution 1 (that is, the FAM concentration was 10 µM and the DMSO concentration was 0.5% in the culture solution in the well).

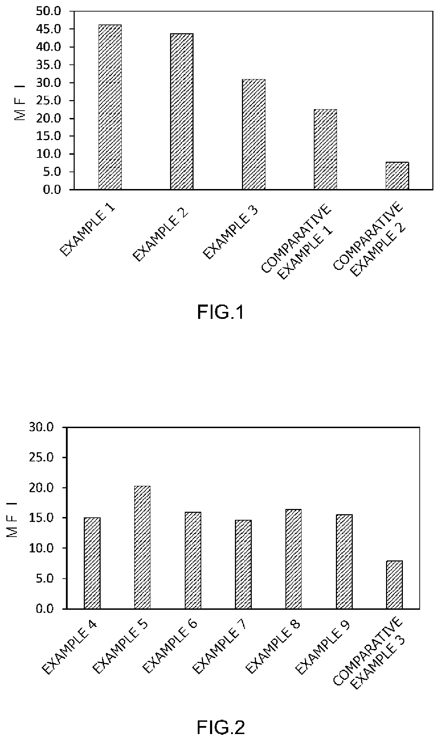

The results obtained from Examples 1 to 9 and Comparative Examples 1 to 3 are shown in Tables 1 and 2 and Figs. 1 and 2. Figs. 1 and 2 show histograms measured by the aforementioned flow cytometer. In Figs. 1 and 2, the vertical axis represents the number of cells and the horizontal axis represents the fluorescence intensity. Note that the tests in Table 1 and Table 2 are independently performed in different days.

### [Table 1]

**Table 1**

| | Structure of construct (additive) | SEQ ID No. of peptide fragment | MFI |
|---|---|---|---|
| Example 1 | Ac-KKRTLRKSARKKR-FAM | SEQ ID No.: 1 | 46.2 |
| Example 2 | Ac-KKRTLRKSASARKKR-FAM | SEQ ID No.: 2 | 43.7 |
| Example 3 | Ac-KKRTLRKSNSNRKKR-FAM | SEQ ID No.: 3 | 31.0 |
| Comparative Example 1 | Ac-KKRTLRKSNRKKR-FAM | SEQ ID No.: 10 | 22.5 |
| Comparative Example 2 | FAM | - | 7.70 |

### [Table 2]

**Table 2**

| | Structure of construct (additive) | SEQ ID No. of peptide fragment | MFI |
|---|---|---|---|
| Example 4 | Ac-KKRTLRKSASNRKKR-FAM | SEQ ID No.: 4 | 15.0 |
| Example 5 | Ac-KKRTLRKSNSARKKR-FAM | SEQ ID No.: 5 | 20.3 |
| Example 6 | Ac-KKRTLRKSNSASARKKR-FAM | SEQ ID No.: 6 | 15.9 |
| Example 7 | Ac-KKRTLRKSASASNRKKR-FAM | SEQ ID No.: 7 | 14.6 |
| Example 8 | Ac-KKRTLRKSNSNSNRKKR-FAM | SEQ ID No.: 8 | 16.4 |
| Example 9 | Ac-KKRTLRKSNSNSNSNRKKR-FAM | SEQ ID No.: 9 | 15.5 |
| Comparative Example 3 | FAM | - | 7.88 |

As shown in Tables 1 and 2 and Figs. 1 and 2, the values of MFI in Examples 1 to 9 were higher than or equal to the value of MFI in Comparative Example 1. In addition, the value of MFI was higher in Examples 1 to 9 than in Comparative Examples 2 to 3. That is to say, it is understood that more FAM was transferred to the inside of the cells by the peptide fragments including the amino acid sequences set forth in SEQ ID No.: 1 to SEQ ID No.: 9. Therefore, it is understood that the peptide fragments including the amino acid sequences set forth in SEQ ID No.: 1 to SEQ ID No.: 9 have the cell membrane permeability and the foreign substance can be transferred from outside the cell to the inside.

Although the detailed data is not shown, the present inventor's examination has indicated that the cytotoxicity in the peptide fragments including the amino acid sequences set forth in SEQ ID No.: 1 to SEQ ID No.: 9 was equal to or suppressed to be lower than the cytotoxicity in the existing peptide fragment (SEQ ID No.: 10).

Although the detailed data is not shown, the present inventor's examination has indicated that the construct including the peptide fragments including the amino acid sequences set forth in SEQ ID No.: 1 to SEQ ID No.: 9 and the polypeptide, the nucleic acid, or the drug as the foreign substance can be transferred efficiently from outside the eukaryotic cell to the inside of the cell.

The specific examples of the art disclosed herein have been described above in detail; however, these are just examples and will not limit the scope of claims. The art described in the scope of claims includes those in which the specific examples given above are variously modified and changed.

In the art disclosed herein, the components and the processes described herein can be omitted as appropriate or combined as appropriate unless any particular problem occurs. The present specification includes the disclosure according to the items given below.
Item 1: The peptide fragment including the A part, the B part, and the C part from the N-terminus of the peptide, in which the A part is the amino acid sequence set forth in SEQ ID No.: 11 (KKRTLRK), the C part is the amino acid sequence set forth in SEQ ID No.: 12 (RKKR), and the B part is positioned between the A part and the C part and is the amino acid sequence in which one unit, two units, three units, or four units are arranged from the group consisting of SA and SN (however, the sequence in which the B part includes only SN is excluded).
Item 2: The peptide fragment according to Item 1, in which the B part is the amino acid sequence in which the amino acid sequence set forth in SA is continuous for one unit, two units, or three units.
Item 3: The peptide fragment according to Item 1, including any of the following amino acid sequences:
   KKRTLRKSARKKR (SEQ ID No.: 1);
   KKRTLRKSASARKKR (SEQ ID No.: 2);
   KKRTLRKSASNRKKR (SEQ ID No.: 3);
   KKRTLRKSNSARKKR (SEQ ID No.: 4);
   KKRTLRKSNSASARKKR (SEQ ID No.: 5);
   KKRTLRKSASASNRKKR (SEQ ID No.: 6);
   KKRTLRKSNSNRKKR (SEQ ID No.: 3);
   KKRTLRKSNSNSNRKKR (SEQ ID No.: 8); and
   KKRTLRKSNSNSNSNRKKR (SEQ ID No.: 9).
Item 4: The construct including: the peptide fragment; and the foreign substance that is bonded to the N-terminus and/or the C-terminus of the peptide fragment, in which the peptide fragment includes the A part, the B part, and the C part from the N-terminus, the A part is the amino acid sequence set forth in SEQ ID No.: 11 (KKRTLRK), the C part is the amino acid sequence set forth in SEQ ID No.: 12 (RKKR), and the B part is positioned between the A part and the C part and is the amino acid sequence in which one unit, two units, three units, or four units are arranged from the group consisting of SA and SN (however, the sequence in which the B part includes only SN is excluded).
Item 5: The construct according to Item 4, in which the B part is the amino acid sequence in which the amino acid sequence set forth in SA is continuous for one unit, two units, or three units.
Item 6: The construct according to Item 4, in which the peptide fragment includes any of the following amino acid sequences:
   KKRTLRKSARKKR (SEQ ID No.: 1);
   KKRTLRKSASARKKR (SEQ ID No.: 2);
   KKRTLRKSASNRKKR (SEQ ID No.: 3);
   KKRTLRKSNSARKKR (SEQ ID No.: 4);
   KKRTLRKSNSASARKKR (SEQ ID No.: 5);
   KKRTLRKSASASNRKKR (SEQ ID No.: 6);
   KKRTLRKSNSNRKKR (SEQ ID No.: 3);
   KKRTLRKSNSNSNRKKR (SEQ ID No.: 8); and
   KKRTLRKSNSNSNSNRKKR (SEQ ID No.: 9).
Item 7: The construct according to Items 4 to 6, in which the foreign substance is at least one kind of organic compound selected from the group consisting of a polypeptide, a nucleic acid, a dye, and a drug.
Item 8: The construct according to Items 4 to 7, in which the foreign substance is disposed at the C-terminus of the peptide fragment.
Item 9: The method for transferring the target foreign substance in vitro from outside the eukaryotic cell to the inside of the cell, the method including: (1) the step of preparing the construct according to any one of Items 4 to 8; and (2) the step of supplying the construct into the sample including the target eukaryotic cell.
Item 10: The method according to Item 9, in which the eukaryotic cell to which the construct is to be transferred is the mammalian cell.

### [Industrial Applicability]

The present disclosure provides the peptide fragment that can transfer the target foreign substance from outside the eukaryotic cell (in particular, various animal cells typified by human and nonhuman mammalian cells that do not have a cell wall) to the inside of the cell, and the construct including the peptide fragment. By using such a construct, the target foreign substance can be effectively transferred into the target cell, and biological tissues such as organs and cells to which the foreign substance has been transferred can be obtained. In addition, by utilizing the peptide fragment or the construct disclosed herein in techniques of drug delivery, therapeutic agents for various diseases (for example, therapeutic agents for ophthalmic treatment or the like) can be provided. In addition, the peptide fragment or the construct disclosed herein can be used as an active ingredient or an additive in medicine for external use (for example, eye drops or the like).

## Claims

1. A peptide fragment comprising an A part, a B part, and a C part from an N-terminus of a peptide, wherein
the A part is an amino acid sequence set forth in SEQ ID No.: 11 (KKRTLRK),
the C part is an amino acid sequence set forth in SEQ ID No.: 12 (RKKR), and
the B part is positioned between the A part and the C part and is an amino acid sequence in which one unit, two units, three units, or four units are arranged from the group consisting of SA and SN (however, a sequence in which the B part includes only one SN unit is excluded).

2. The peptide fragment according to claim 1, wherein the B part is the amino acid sequence in which an amino acid sequence set forth in SA is continuous for one unit, two units, or three units.

3. The peptide fragment according to claim 1, wherein the peptide fragment including the A part, the B part, and the C part includes any of the following amino acid sequences:
KKRTLRKSARKKR (SEQ ID No.: 1);
KKRTLRKSASARKKR (SEQ ID No.: 2);
KKRTLRKSNSNRKKR (SEQ ID No.: 3);
KKRTLRKSASNRKKR (SEQ ID No.: 4);
KKRTLRKSNSARKKR (SEQ ID No.: 5);
KKRTLRKSNSASARKKR (SEQ ID No.: 6);
KKRTLRKSASASNRKKR (SEQ ID No.: 7);
KKRTLRKSNSNSNRKKR (SEQ ID No.: 8); and
KKRTLRKSNSNSNSNRKKR (SEQ ID No.: 9).

4. A construct comprising:
a peptide fragment; and
a foreign substance that is bonded to an N-terminus and/or a C-terminus of the peptide fragment, wherein
the peptide fragment includes an A part, a B part, and a C part from the N-terminus,
the A part is an amino acid sequence set forth in SEQ ID No.: 11 (KKRTLRK),
the C part is an amino acid sequence set forth in SEQ ID No.: 12 (RKKR), and
the B part is positioned between the A part and the C part and is an amino acid sequence in which one unit, two units, three units, or four units are arranged from the group consisting of SA and SN (however, a sequence in which the B part includes only SN is excluded).

5. The construct according to claim 4, wherein the B part is the amino acid sequence in which an amino acid sequence set forth in SA is continuous for one unit, two units, or three units.

6. The construct according to claim 4, wherein the peptide fragment including the A part, the B part, and the C part includes any of the following amino acid sequences:
KKRTLRKSARKKR (SEQ ID No.: 1);
KKRTLRKSASARKKR (SEQ ID No.: 2);
KKRTLRKSNSNRKKR (SEQ ID No.: 3);
KKRTLRKSASNRKKR (SEQ ID No.: 4);
KKRTLRKSNSARKKR (SEQ ID No.: 5);
KKRTLRKSNSASARKKR (SEQ ID No.: 6);
KKRTLRKSASASNRKKR (SEQ ID No.: 7);
KKRTLRKSNSNSNRKKR (SEQ ID No.: 8); and
KKRTLRKSNSNSNSNRKKR (SEQ ID No.: 9).

7. The construct according to claim 4, wherein the foreign substance is at least one kind of organic compound selected from the group consisting of a polypeptide, a nucleic acid, a dye, and a drug.

8. The construct according to claim 4, wherein the foreign substance is bonded to the C-terminus of the peptide fragment.

9. A method for transferring a target foreign substance in vitro from outside a eukaryotic cell to an inside of the cell, the method comprising:
(1) a step of preparing the construct according to any one of claims 4 to 7; and
(2) a step of supplying the construct into a sample including a target eukaryotic cell.

10. The method according to claim 9, wherein the eukaryotic cell to which the construct is to be transferred is a mammalian cell.
